# EUROPEAN PATENT SPECIFICATION

(11) **EP 2 593 119 B1**
(45) Date of publication and mention of the grant of the patent: **04.11.2015**
(21) Application number: 11745921.4
(22) Date of filing: 01.07.2011
(51) Int. Cl.: A61K 36/48, A61P 11/14

(54) **POLYSACCHARIDE POLYMER FROM THE SEEDS OF THE TAMARIND TREE FOR USE IN TREATING DRY COUGH**
POLYSACCHARID-POLYMERE AUS DEN SAMEN DER TAMARINDE ZUR BEHANDLUNG VON REIZHUSTEN
POLYMÈRE DE POLYSACCHARIDE PROVENANT DE GRAINES DU TAMARINIER POUR UNE UTILISATION DANS LE TRAITEMENT D'UNE TOUX SÈCHE

(30) Priority: 12.07.2010 EP 10007151
(43) Date of publication of application: 22.05.2013
(73) Proprietor: Zambon S.p.A., 20091 Bresso MI (IT)
(72) Inventor: MELLONI, Elsa, 20162 Milano (IT); SARDINA, Marco, 21040 Gerenzano (Varese) (IT)
(74) Representative: Allaix, Roberto
(86) International application number: PCT/EP2011/003284
(87) International publication number: WO 2012/007113

(56) References cited:
- WO-A1-97/28787
- WO-A1-2004/113390
- WO-A1-2006/069213
- WO-A2-2009/040847
- MURRAY R ET AL: "An outbreak of weaver's cough associated with tamarind seed powder", BRITISH JOURNAL OF INDUSTRIAL MEDICINE, BRITISH MEDICAL ASSOCIATION, LONDON, GB, vol. 14, no. 2, 1 April 1957 (1957-04-01), pages 105-110, XP009151988, ISSN: 0007-1072
- TUFFNELL P G ET AL: "An investigation into the acute respiratory reaction to the inhalation of tamarind seed preparations", BRITISH JOURNAL OF INDUSTRIAL MEDICINE, BRITISH MEDICAL ASSOCIATION, LONDON, GB, vol. 14, no. 4, 1 October 1957 (1957-10-01), pages 250-252, XP009151989, ISSN: 0007-1072

## Description

### FIELD OF THE INVENTION

The present invention relates to the use of polysaccharide polymer obtained from the seeds of the tamarind tree for treating dry cough.

Especially, the present invention relates to treatment of the dry cough in children.

### DESCRIPTION OF THE INVENTION

Dry cough often occurs at the end of viral infections affecting the upper respiratory airways such as a cold and flu, or after the exposure to a variety of irritating factors including allergens, dust, smoke, cleaning agents, chemical air pollutants and contaminants, prolonged stay in dry environments and physical traumas.

Dry cough typically does not last longer than 2-3 weeks.

Typically, a person suffering from dry cough experiences an unpleasant feeling of burning and dryness of the high respiratory airways and an uncomfortable tickling in the throat, which usually begins the attacks of cough.

In most cases, dry cough is mild throughout the day and often gets more intense at night, preventing a person from sleeping or resting. Sometimes dry cough occurs only at night, after the person goes to sleep.

Dry cough is very common in children. Children who cough during the night may have problems to sleep, and such problems may involve also their parents Furthermore, if left untreated for a longer period of time, dry cough might complicate possibly resulting in chronic dry cough, or also in bacterial infections that complicate the clinical outcome, prolonging the time to recovery.

A lot of traditional homemade remedies and over-the-counter medicines are commonly used as first line treatment for dry cough.

Traditional homemade remedies include, for example, fresh ginger, saline solution and honey.

Common over-the-counter pharmacological remedies include, for example, dextromethorphan and diphenhydramine.

However, traditional homemade remedies, generally recognized as safe, are often ineffective in relieving the cough, whereas over-the-counter remedies are known to potentially cause side effects, such as allergic reactions, effects on sleep or hallucinations and they are not recommended for children, especially children under six years of age, because there is no clear evidence of their efficacy.

Thus, there is a need for an effective treatment of dry cough, without the adverse side-effects of the medicaments conventionally used for this purpose. The control of children cough during the night is particularly awaited, so achieving undisputed beneficial effect also on parental sleep.

Tamarind tree is widespread in India, Africa and in the South East Asia. In Middle Eastern countries, tamarind juice from the tamarind fruit can be a drink prepared by infusing dried tamarind pulp. Tamarind can also be useful for the preservation of food products and as a sauce in recipes.

The tamarind seed finds various applications, once ground to the powder form (known as "tamarind gum" or "tamarind kernel powder"). Commercially available tamarind kernel powder can be employed as thickener and a sizing agent in textile and paper industries; and as thickening, gelling, stabilizing and binding agent in food and pharmaceutical industries.

General properties, chemical composition and chemical structure of tamarind kernel powder and of tamarind seed polysaccharide can be found in: Gupta V, Puri R, Gupta S, Jain S, Rao GK.; Tamarind kernel gum: An upcoming natural polysaccharide. Syst Rev Pharm; 2010;1:50-4.

It has now surprisingly found that a polysaccharide polymer obtained from the seeds of the tamarind tree provides dry cough relief and maintains the relief for an extended period of time that is long enough to allow, for example, the adults and children to fall asleep and enjoy a satisfactory sleep time.

It is therefore a first object of the present invention a polysaccharide polymer obtained from the seeds of the tamarind tree (tamarind seed polysaccharide), for use in treating dry cough.

Tamarind seed polysaccharide is a nonionic, neutral, branched polysaccharide comprising a cellulose-like backbone substituted by xylose (α 1→6) and galacto (β 1→2)xylose (α 1→6) substituents (Lang P. et al., Macromolecules, 1993, 26, 3992-3998).

Tamarind seed polysaccharide suitable for the preparations according to the present invention may be obtained according to any method known in the art, preferably following the methods described in WO 97/28787.

Tamarind seed polysaccharide according to the invention is particularly effective for treating dry cough in children, especially at night, when cough is particularly bothersome because it disrupts sleep.

To achieve the desired effect, a polysaccharide polymer obtained from the seeds of the tamarind tree according to the invention is locally administered to the mucous membranes of the high respiratory airways of a subject suffering from dry cough as an aerosolized liquid preparation, by suitable commercially available inhalation devices.

It is therefore another object of the present invention a preparation in the form of a liquid dosage for aerosolized topical application, which comprises a polysaccharide polymer obtained from the seeds of the tamarind tree, said preparation being useful for treating dry cough, especially dry cough in children.

The preparation according the invention contains from 0.25 to 0.5 w/v% (hereunder the unit "w/v%" is referred to as "%" for simplicity) of the polysaccharide polymer obtained from the seeds of the tamarind tree. Preferably, the preparation according the invention contains 0.3% of the polysaccharide polymer obtained from the seeds of the tamarind tree.

The preparation according to the invention can further comprise pH adjusting agents well known to a person skilled in the art, including pharmaceutically acceptable acids or bases and buffering agents. For example, the acids may include one or more inorganic mineral acids such as hydrochloric, hydrobromic, sulfuric, phosphoric and nitric acid, or one or more organic acids such as acetic, succinic, tartaric, ascorbic, citric, glutamic, benzoic, methanesulphonic, ethanesulfonic and trifluoroacetic acid. The bases may include inorganic bases and organic bases, such as alkaline carbonate, alkaline bicarbonate, alkaline earth metal carbonate, alkaline hydroxide and alkaline earth metal hydroxide. For example, the inorganic base may be an alkaline hydroxide such as lithium hydroxide, potassium hydroxide, cesium hydroxide or sodium hydroxide; an alkaline carbonate may be calcium carbonate or sodium carbonate; an alkaline bicarbonate may be sodium bicarbonate. For example, buffering agents include a phosphate buffer such as sodium dihydrogen phosphate commonly termed monosodium phosphate (NaH₂PO₄) and disodium hydrogen phosphate commonly termed disodium phosphate (Na₂HPO₄), an acetate buffer (sodium acetate-acetic acid system) a citrate buffer (sodium citrate-citric acid system) and mixtures thereof.

In a particular aspect of the present invention the buffering agent is preferably selected from monosodium phosphate (NaH₂PO₄), disodium phosphate (Na₂HPO₄) or mixture thereof, and sodium citrate-citric acid system.

The type and concentration of the pH adjusting agents suitable for use in the disclosed preparation would be dependent on the desired pH value which ranges within 6.0 to 8.0, preferably within 6.8 and 7.5.

The isotonicity of the preparation vehicle is preferably adjusted by sodium chloride. The local administration to the mucous membranes of the high respiratory airways of a subject suffering from dry cough of the preparation according to the invention is carried out by an aerosol delivery system; the aerosolized liquid preparation is therefore sufficiently mobile to coat a wide area of the mucosal surface of the upper respiratory tract, thus providing effective relief of dry cough.

Preferably, the aerosol delivery system is a commercially available nebulizer, such as a jet nebulizer, which accepts the liquid preparation and transforms said liquid preparation into fine droplets, so that it can be easily inhaled through a mouthpiece or a face mask.

As an example, when the jet nebulizer is turned on, a jet of air is forced through the preparation according to the invention, turning it into fine droplets which are moved along a tube. The subject inhales the preparation, through a mouthpiece or a face mask. A face mask is preferably used for pediatric subjects.

The preparation according to the invention can be administered to a subject suffering from dry cough on as-needed or as-desired basis, for example, at least once daily and preferably multiple, for example 2 or 3 times daily.

The amount of the preparation administered to a subject may be dependent on a variety of factors, including the general quality of health of the subject, age, gender, weight, or severity of dry cough. For example, from 6 to 18 mg/day of tamarind seed polysaccharide can be administered to a subject in need thereof.

A process for preparing a preparation as described above can be carried out according to methods well known to a person skilled in the art; for example, said process comprises dissolving the tamarind seed polysaccharide in purified water such as water for injections and adding a pH adjusting agent until the desired pH is reached.

As used herein, the singular forms "a," "an" and "the" include plural referents unless the context clearly states otherwise. Thus, for example, reference to "a polysaccharide polymer obtained from the seeds of the tamarind tree" includes mixtures of polysaccharide polymers obtained from the seeds of the tamarind tree. As used herein, the term "high respiratory tract" includes nasal, sinus, pharyngeal, laryngeal, tracheal, and bronchial compartments.

As used herein, the term "inhalation" or "administration by inhalation" refers to the introduction into the respiratory organs, especially into and/or via the airways, preferably into and/or via the nasal cavity and oral cavity.

As used herein, the term "nebulizer" includes jet nebulizers.

As used herein, the term "polysaccharide polymer obtained from the seeds of the tamarind tree", interchangeable with the term "tamarind seed polysaccharide" and abbreviated as "TSP", means any polysaccharide-enriched fraction obtainable from tamarind kernel powder currently available on the market. A partially purified polysaccharide fraction of tamarind gum is sold, for instance, by Dainippon Pharmaceutical Co. LTD of Osaka, Japan, under the trade name Glyloid^{®}. For the purpose of the present invention, however, the concerned polysaccharide is preferably obtained following any method known in the art to give a practically pure tamarind seed polysaccharide from commercial tamarind gum sources.

As used herein, the term "preparation" is intended in a broader sense to include not only all conventional pharmaceutical preparations but also preparations useful as diet supplements, medical devices and food additives.

As used herein, the term "subject" refers to a human adult or a child afflicted with a dry cough.

As used herein the term "treating" means relieving, suppressing, reducing, decreasing, delaying, ameliorating, calming or preventing onset, progression, severity, duration, frequency of dry cough and not necessarily indicates a total elimination the same.

Example 1 illustrates some preparations according to the invention.

### Example 1

The necessary amount of tamarind seed polysaccharide (TSP) was weighted into a suitable vessel, water for injection was added up to the final volume (100 percent) until complete dissolution of the TSP. The isotonicity of the preparation was adjusted by NaCl and pH adjusting agents were then added to maintain the pH within physiological pH (between 6.8 and 7.5). The so obtained preparation had the following composition:

| | |
|---|---|
| TSP | 0.3% |
| NaCl | 0.9%, |
| Na₂HPO₄ | 0.9%, |
| NaH₂PO₄ | 0.09% |
| Water for injection | to 100 ml |

Following analogous procedure, the below preparation was obtained:

| | |
|---|---|
| TSP | 0.3% |
| NaCl | 0.9%, |
| Citric acid | 0.03%, |
| Sodium citrate | 0.5% |
| Water for injection | to 100 ml |

Example 2 illustrates the use in one adult subject of the first preparation of Example 1.

### Example 2

One subject with cough following inflammation of the upper airways was selected to test the preparation of Example 1.

The preparation was locally administered by aerosol inhalation carried out by the nebulizer Fluirespira Professional (Zambon).

Application of the preparation was more effective than saline solution in alleviating the cough.

Furthermore the subject declared a pleasant hydrating and soothing sensation and relief of sensory discomfort in the upper airways.

## Claims

1. A polysaccharide polymer obtained from the seeds of the tamarind tree, for use in treating dry cough, wherein the polymer is to be locally administered to the mucous membranes of the high respiratory airways of a subject suffering from dry cough as an aerosolized liquid preparation.

2. The polysaccharide polymer for use according to claim 1, wherein the subject suffering from dry cough is a child.

3. A preparation in the form of a liquid dosage for aerosolized topical application, which comprises a polysaccharide polymer obtained from the seeds of the tamarind tree, for use in treating dry cough.

4. A preparation for use according to claim 3, which is to be locally administered to the mucous membranes of the high respiratory airways of a subject suffering from dry cough.

5. A preparation for use according to claim 4, wherein the polysaccharide is obtained by purification from commercial tamarind gum sources.

6. A preparation for use according to claim 4, which contains from 0.25 to 0.5% of the polysaccharide polymer obtained from the seeds of the tamarind tree.

7. A preparation for use according to claim 6, which contains 0.3% of the polysaccharide polymer obtained from the seeds of the tamarind tree.

8. A preparation for use according to claim 4, which further comprises a pH adjusting agent.

9. A preparation for use according to claim 8, wherein the pH adjusting agent is a buffering agent selected from monosodium phosphate (NaH₂PO₄), disodium phosphate (Na₂HPO₄), sodium citrate-citric acid system or mixture thereof.

## Patentansprüche

1. Aus den Samen des Tamarindenbaumes erhaltenes Polysaccharidpolymer zur Verwendung für die Behandlung von trockenem Husten, wobei das Polymer als aerosolierte flüssige Zusammensetzung lokal auf die Schleimhäute der oberen respiratorischen Atemwege eines an trockenem Husten leidenden Patienten zu applizieren ist.

2. Polysaccharidpolymer zur Verwendung nach Anspruch 1, wobei der an trockenem Husten leidende Patient ein Kind ist.

3. Zusammensetzung in Form einer flüssigen Dosierungsform zur aerosolierten topischen Anwendung, die ein aus den Samen des Tamarindenbaumes erhaltenes Polysaccharidpolymer enthält, zur Verwendung für die Behandlung von trockenem Husten.

4. Zusammensetzung zur Verwendung nach Anspruch 3, die lokal auf die Schleimhäute der oberen respiratorischen Atemwege eines an trockenem Husten leidenden Patienten zu applizieren ist.

5. Zusammensetzung zur Verwendung nach Anspruch 4, wobei das Polysaccharid durch Aufreinigung aus handelsüblichen Tamarindengummiquellen erhalten wird.

6. Zusammensetzung zur Verwendung nach Anspruch 4, die von 0,25 bis 0,5 % des aus den Samen des Tamarindenbaumes erhaltenen Polysaccharidpolymers enthält.

7. Zusammensetzung zur Verwendung nach Anspruch 6, die 0,3 % des aus den Samen des Tamarindenbaumes erhaltenen Polysaccharidpolymers enthält.

8. Zusammensetzung zur Verwendung nach Anspruch 4, die außerdem ein Mittel zur Einstellung des pH-Werts enthält.

9. Zusammensetzung zur Verwendung nach Anspruch 8, wobei das Mittel zur Einstellung des pH-Werts eine unter Mononatriumphosphat (NaH₂PO₄), Dinatriumphosphat (Na₂HPO₄), Natriumcitrat-Citronensäuren-System oder deren Mischung ausgewählte Puffersubstanz ist.

## Revendications

1. Polymère de polysaccharide obtenu à partir des graines du tamarinier, pour utilisation dans le traitement de la toux sèche, lequel polymère est destiné à être administré localement aux membranes muqueuses des voies respiratoires supérieures d'un sujet souffrant d'une toux sèche sous la forme d'une préparation liquide en aérosol.

2. Polymère de polysaccharide pour utilisation selon la revendication 1, dans lequel le sujet souffrant d'une toux sèche est un enfant.

3. Préparation sous la forme d'une dose liquide pour application par voie topique en aérosol, qui comprend un polymère de polysaccharide obtenu à partir des graines du tamarinier, pour utilisation dans le traitement de la toux sèche.

4. Préparation pour utilisation selon la revendication 3, qui est destinée à être administrée localement aux membranes muqueuses des voies respiratoires supérieures d'un sujet souffrant d'une toux sèche.

5. Préparation pour utilisation selon la revendication 4, dans laquelle le polysaccharide est obtenu par purification à partir de sources de gomme de tamarin du commerce.

6. Préparation pour utilisation selon la revendication 4, qui contient de 0,25 à 0,5 % du polymère de polysaccharide obtenu à partir des graines du tamarinier.

7. Préparation pour utilisation selon la revendication 6, qui contient 0,3 % du polymère de polysaccharide obtenu à partir des graines du tamarinier.

8. Préparation pour utilisation selon la revendication 4, qui comprend en outre un agent d'ajustement du pH.

9. Préparation pour utilisation selon la revendication 8, dans laquelle l'agent d'ajustement du pH est un agent tampon choisi parmi le phosphate monosodique (NaH₂PO₄), le phosphate disodique (Na₂HPO₄), le système citrate de sodium-acide citrique, et leurs mélanges.
